# EUROPEAN PATENT APPLICATION

(11) **EP 4 338 590 A1**
(43) Date of publication of application: **20.03.2024**
(21) Application number: 22807260.9
(22) Date of filing: 29.03.2022
(51) Int. Cl.: A01N 1/02, C12N 5/073, C12N 5/10

(54) **COLD STORAGE SOLUTION AND STORAGE METHOD FOR STEM CELLS**

(30) Priority: 11.05.2021 JP 2021080546
(71) Applicant: BMG INCORPORATED, Kyoto-shi, Kyoto 601-8023 (JP)
(72) Inventor: AIZAWA Akira, Kyoto-shi, Kyoto 601-8023 (JP); HYON Suong-Hyu, Kyoto-shi, Kyoto 601-8023 (JP); HYON Woogi, Kyoto-shi, Kyoto 601-8023 (JP)
(74) Representative: Global IP Europe Patentanwaltskanzlei
(86) International application number: PCT/JP2022/015708
(87) International publication number: WO 2022/239556

(57) **Abstract**

Provided are a cold storage method and a cold storage liquid that are suitable for non-freezing refrigerated storage of human or animal stem cells such as iPS cells or embryos in a non-frozen state. According to an embodiment, the non-freezing refrigerated storage liquid is: a mixture liquid (HTMα, HTMx2c13), in which MEM-alpha (Minimum Essential Medium Eagle (MEM) Alpha Modification) is mixed with a UW solution (UW_sln; BELZER UW (registered trademark) COLD STORAGE SOLUTION), or a modified UW solution (polymer component being changed to PVA), are mixed in a ratio of about 1/1 to 1/2; or a storage liquid having a composition equivalent to the mixture liquid, especially in respect of concentrations of potassium ions and sodium ions. And, arousal period(s) of maintaining a temperature of 30°C to 37°C is inserted at each of two to five days, into a non-freezing refrigerated storage period, which is made at 2°C to 8°C for 2 to 10 days or 4 to 20 days.

## Description

The present invention relates to a storage liquid for preserving cells, tissues, organoids, organs, etc. in a non-frozen state. In particular, it relates to a refrigerated storage liquid for storage at a temperature lower than room temperature, for example, 2 to 15 °C, 2 to 8 °C, or 2 to 6 °C. Further, in particular, for storing, by suspending or immersing, pluripotent cells such as human iPS cells and other stem cells, derivatives such as organoids obtained from them, early embryos, etc.

Convenient, efficient and stable storage and supply of stem cells such as iPS and ES cells is an important technology that is indispensable in the fields of basic research and clinical application research or drug discovery research, in regenerative medicine. Cryopreservation of human iPS cells and ES cells has many problems such as: low survival rate after storage when the slow-freezing cryopreservation method is used; cytotoxicity of DMSO, which is a cryoprotective agent used for cryopreservation; and differentiation inducing capability. Non-freezing low-temperature storage of cells is a very attractive technique as a short-term (for example, 1 day to 2 weeks) to medium-term (for example, half a month to 3 months) preservation method, and in same time would be very advantageous in preservation of differentiation-induced cells and the preservation of organoids when the industrialization of regenerative medicine technology and its application to drug discovery are considered. However, it is difficult to cryopreserve pluripotent stem cells including human iPS cells using UW solution (UW_sln; BELZER UW^{®} COLD STORAGE LIQUID), which is widely used as a cryopreservation liquid for organs. Therefore, it is essential to develop a new refrigerated storage technology.

UW solution is widely used for refrigerated storage of organs used for transplantation such as liver and kidney, and it is practically a de facto standard or gold standard for such. The UW solution was developed in the 1980s and has a rather simple composition having an intracellular-type salt composition of low Na ion content and high K ion content, and containing lactobionat, raffinose, glutathione, hydroxyethyl starch.
Non-patent document 1: The Molecular Biology Society of Japan Program, 41st Molecular Biology Society, November 9, 2018; 3P-0445, Low-temperature-stress and oxidation-stress protection effect by newly developed non-freezing low-temperature storage liquid for human iPS cells; Akira AIZAWA1, Misa KII1, Woogi HYON1, Suong-Hyu HYON2 (1. Bioverde Co., Ltd. 2. Kagoshima National University, Joint Veterinary School)
Non-patent document 2: The 18th Annual Meeting of the Japanese Society for Regeneration and Medical Care; February 22, 2018, P-01-015, Development of a new storage solution with low-temperature stress / oxidative stress protection effect for refrigerated storage of human iPS cells; Akira AIZAWA (Bioverde Co., Ltd.)
Non-patent document 3: COMPARE BELZER UW™ COLD STORAGE LIQUID TO VIASPAN https://bridgetolife.com/compare-belzer-uw-cold-storage-solution-to-viaspan/

However, when to store cells or tissues in a non-freezing refrigerated state, UW solution cannot sufficiently suppress low-temperature storage stress. To solve this problem, the inventors of the present invention have hypothesized that it was important to adopt an intermediate between: the intracellular type compositions represented by the UW solution; and extracellular type compositions having high Na ion content and low K ion content that are common in generally used culture media. In addition, we have conceived and idea that the addition of macromolecules such as proteins is important on contrary to organ preservation. Thus, the inventors has investigated storage liquids that have compositions at an intermediate between the UW solution and the cell culture media.

There are few reports on the low-temperature storage medium for cells. There are only a few reports of storage by using recently developed HTS FRS (Hypothermosol^{®} FRS; BioLife Solutions). It has been reported that this HTS FRS can store human skin fibroblasts and smooth muscle cells at the low temperature for 3 to 5 days, but there is no report on the non-freezing refrigerated storage of pluripotent cells such as human iPS cells.

As mentioned in to-be published PCT/JP2020/41313, while trying a wide variety of combinations, the inventors of the present invention have adopted mixing, at a ratio of about 1/1 to 1/2, MEM-alpha (Minimum Essential Medium Eagle (MEM) Alpha Modification) with the above UW solution. The MEM-alpha is a modification based on MEM medium and contains non-essential amino acids, sodium pyruvate, lipoic acid, biotin and vitamin B12. By adopting such improved non-freezing refrigerated storage liquid, is has been known to maintain some degree of viability of the human iPS cells or the like for a period of several days or more.

While trying to make further improvements, the inventors came up with a very originative idea of whether it would be possible to adopt a "hibernation model", which is seen in rodents (Muridae) or the like. In hibernating rodents, short arousal periods are essential between low-temperature periods. During the arousal period, the rodent returns to a state of around 37°C of body temperature for several hours so as to take a food, once in several days to once a week; and then, returns to a state of hypothermia.

While adopting the above-mentioned improved non-freezing refrigerated storage liquid, the inventors attempted inserting arousal period(s) during the non-freezing refrigerated storage period. In other words, the following was attempted : short periods of returning to a culture condition at near 37°C are inserted into a non-freezing refrigerated storage period in a way similar with a manner that: short arousal periods are repeated at certain intervals during hibernation, which is made at hypothermia from around 20°C to less than 10°C. Subsequently, the inventors have verified effect of such inserting, through experiments, so as to complete the invention.

Thus, in preferred embodiments, the non-freezing refrigerated storage is made according to (i) to (iii) below.
(i) Adopted non-freezing refrigerated storage liquid is a mixture liquid, in which MEM-alpha and UW solution or modified UW solution (polymer component is changed to PVA) are mixed at a ratio of about 1/1 to 1/2, or is an equivalent storage liquid that is equivalent to the above mixture liquid in terms of concentrations of potassium and sodium ions and their mutual ratio.
(ii) Arousal periods are inserted at regular (fixed) or irregular intervals to the non-freezing refrigerated storage, which is made, for example, at 2°C to 10°C for 2 days to 10 days, 3 to 15 days, or 4 to 20 days.
(iii) The arousal periods may be set as follows:
   - Length of each arousal period: 0.5 to 10 hours, especially 1 to 8 hours.
   - Temperature during each arousal period: 37°C or lower, 30°C or higher, 32°C or higher, 33°C or higher, 34°C or higher, or 35°C or higher.
   - Arousal period interval: 2 to 8 days, especially 2 to 5 days, for example 3 to 4 days.

According to a preferred embodiment of the present invention, refrigerated storage at 2°C to 10°C for example is carried out by standing in a refrigerator or in a refrigerating chamber; and the insertion of an arousal period is performed by transferring to a normal incubator and under normal cell culture conditions (typically, at 37°C and 5% CO₂).

In addition, the insertion of the arousal period should be performed at regular intervals and for a constant length, for example, every 3 days, every 2 days, or every 4 days, and for a constant time set in a range of 1 to 6 hours. It is also possible, for example, to insert the first arousal period 3 days after the start of culture, and then insert the second arousal period at an interval of 4 days. It is also possible to set the first arousal period to 3 hours and the second arousal period to 4 hours.

The MEM (Eagle's minimal essential medium) medium such as MEM-alpha contains the following components (1) to (4).
(1) Amino acids (L-arginine, L-cystine, L-glutamine, L-histidine, L-isoleucine, L-leucine, L-lysine, L-phenylalanine, L-threonine, L-tryptophan, L-tyrosine, L -Valin),
(2) Salts (calcium chloride, potassium chloride, magnesium sulfate, sodium chloride, sodium dihydrogen phosphate),
(3) D-glucose and
(4) Vitamins (folic acid, nicotinamide, riboflavin, B12, choline, inositol, pantothenic acid, pyridoxal phosphate, thiamine).

On the other hand, the UW solution is a Ca²⁺-free solution having an intracellular fluid composition, and contains: lactobionic acid and hydroxy-ethyl starch (HES) for adjusting osmotic pressure and suppressing cell expansion; adenosine as anti-inflammatory and vascular relaxation agent and as a precursor of ATP; glutathione and alloprinol for antioxidants; and a phosphate buffer as a buffer component.

The non-freezing refrigerated storage liquid according to a preferred embodiment of the present invention has features A1 to A6 or A1 to A9 at below.

A1. Content of potassium ion species is 20 to 90 mmoL/L or 30 to 80 mmoL/L, and the content of sodium ion species is 20 to 90 mmoL/L or 30 to 80 mmoL/L.

A2. Ion-based molar ratio (Na⁺ / K⁺ ratio) of sodium ion species to potassium ion species is 0.5 to 1.5, 0.5 to 1.3, or 0.6 to 1.2.

A3. Trolox or its analog is contained at concentrations of 0.5-10 mM, 1-8 mM or 2-7 mM.

A4. Adenine or a salt or derivative thereof is contained at a concentration of 0.1 to 4.2 mM, 0.1 to 6 mM, 1 to 6 mM, 2 to 5 mM or 3 to 5 mM.

A5. All or all but one, two or three essential amino acids are contained, and the total content of essential amino acids is preferably 50 to 250 mg/L, particularly 50 to 200 mg/L or 80 to 150 mg/L.

A6. Total content of non-essential amino acids is preferably 100 to 500 mg/L, especially 50 to 200 mg/L or 80-150 mg/L.

A7. Content of magnesium ion species (particularly in magnesium sulfate) is 2 to 8 mmoL/L, 2 to 5 mmoL/L or 2-4 mmoL/L, and the calcium content is 0.2 to 1.5 mmoL/L, 0.2 to 1 mmoL/L or 0.3 to 0.8 mmoL/L.

A8. Glucose or other low-molecular-weight polysaccharides (particularly, disaccharides such as maltose, and monosaccharides such as fructose) is contained at a concentration of 0.5 to 10 mmoL/L.

A9. Vitamins at below are contained whereas one, two or three among the below can be omitted.

Folic acid, nicotinamide, riboflavin, B 12, choline, inositol, pantothenic acid, pyridoxal phosphate, and thiamine.

The non-freezing refrigerated storage liquid according to a preferred embodiment of the present invention is a physiological solution, pH of which is set in a range of 7 to 7.5, and comprises:
(i) lactobionic acid or a salt thereof (lactobionate) in terms of lactone, in a range of 30 to 100 mmoL/L (or 10 to 40 g/L),
(ii) raffinose hydrate in a range of 10 to 30 mmoL/L (or 5 to 20 g/L),
(iii) alloprinol in a range of 0.3 to 1 mmoL/L (or 0.05 to 0.1 g/L),
(iv) glutathione (total glutathione) in a range of 1 to 3 mmoL/L,
(v) adenosine in a range of 2 to 10 mmoL/L (or 0.3 to 0.9 g/L),
(vi) lipoic acid in a range of 0.05-1 µmoL/L (or 0.03 to 0.2 mg/L),
(vii) sodium pyruvate in a range of 0.1 to 1 mmoL/L (or 10 to 100 mg/L),
(viii) glucose in a range of 0.5 to 10 mmoL/L (or 100 to 1000 mg/L),
(ix) ascorbic acid in a range of 0.03 to 0.3 mmoL/L, and vitamin E or its water-soluble analog/derivative in a range of 1 to 10 mM,
(x) adenine or its salt or derivative, in a range of 0.1 to 4.2 mM,
(xi) other vitamins or water-soluble analog/derivative thereof,
(xii) essential amino acids in a range of 50 to 200 mg/L in total,
(xiii) non-essential amino acids in a range of 100 to 500 mg/L in total,
(xiv) potassium ion species in a range of 30 to 80 mmoL/L, and
(xv) sodium ion species in a range of 20 to 90 mmoL/L.

This non-freezing refrigerated storage liquid preferably further comprises:
(xv) hydroxyethyl starch in a range of 20 to 50 g/L; and/or
(xvi) ribonucleoside in a range of 4 to 40 mg/L, and/or
(xvii) deoxyribonucleoside in a range of 4-40 mg/L; and/or
(xviii) potassium dihydrogen phosphate and sodium dihydrogen phosphate, in a range of 10 to 25 mmoL/L.

In occasions of non-freezing refrigerated storage of pluripotent cells such as human iPS cells, it is able to maintain a high viability maintenance effect and proliferative ability after recultivation.
FIG. 1 is a graph showing changes in growth rate in course of lapse of the non-freezing refrigerated storage period when continuously stored at 4°C using the improved refrigerated storage liquid that is same with that used in the embodiment.
FIG. 2 is a schematic diagram illustrating main points of the procedure for non-freezing refrigerated storage and evaluation by refrigerated storage with arousal-period insertion (lower row) according to the embodiment and by continuous refrigerated storage without arousal-period insertion (upper row).
FIG. 3 is a column chart showing influence of length of the arousal period when the arousal period is inserted after 3 days, in course of the 6-day refrigerated storage period.
FIG. 4 is a set of fluorescence micrographs, as converted to gray scale, showing dead cells immediately after the end of refrigerated storage, and after 1 hour of recovery culture; in course of the refrigerated storage, by which the results of the FIG. 3 has been obtained.
FIG. 5 is a graph summarizing the results of 12 days of refrigerated storage using two types of improved refrigerated storage liquids and two types of conventionally common refrigerated storage liquids. This graph shows and summarizes occasions when three-hour arousal periods were inserted every 3 days (Hib) and occasions when no arousal period is inserted.
FIG. 6 is a column chart showing effects of inserting the arousal periods, in course of the non-freezing refrigerated storage of mouse embryos.
FIG. 7 is a graph summarizing the results of 12 days of refrigerated storage of mouse embryos in an improved refrigerated storage liquid. Tis graph shows and summarizes: occasions when three-hour arousal periods were inserted every 3 days (Hib); and occasions when no arousal period is inserted.

The non-freezing refrigerated storage liquid according to a preferred embodiment of the present invention is a physiological buffer solution, pH of which is set to 7 to 7.5, comprises (i) to (vi) at below.
(i) Lactobionic acid or a salt thereof (lactobionate) in terms of lactone 20-100 mmoL/L, 30-100 mmoL/L, 30-80 mmoL/L or 30-60 mmoL/L, or 10-50 g/L, 10-40g/L, 15-35g/L or 20-30g/L.
(Ii) Raffinose hydrate 10-40 mmoL/L, 10-30 mmoL/L or 10-20 mmoL/L, or 5-20 g/L or 8-15 g/L.
(iii) Allopurinol 0.3 to 2 mmoL/L, 0.3 to 1 mmoL/L or 0.4 to 0.8 mmoL/L, or 0.03 to 0.15 g/L, 0.05 to 0.1 g/L or 0.06 to 0.08 g/L.
(iv) Glutathione (total glutathione) 1-3 mmoL/L or 1.5-2.5 mmoL/L, or 0.3-1 g/L, 0.4-0.9 g/L or 0.4-0.8 g/L.
(v) Adenosine 2-10 mmoL/L, 2-8 mmoL/L or 2-5 mmoL/L, or 0.5-1.5 g/L or 0.5-1.3 g/L.
(vi) Lipoic acid 0.05 to 1 µmoL/L, 0.1 to 0.5 µmoL/L or 0.2 to 0.4 µmoL/L, or 0.03 to 0.2 mg/L or 0.05 to 0.1 mg/L.
(vii) Sodium pyruvate 0.1 to 1 mmoL/L, 0.1 to 0.7 mmoL/L or 0.2 to 0.5 mmoL/L, or 10 to 100 mg/L or 20 to 50 mg/L.
(viii) Glucose 0.5 to 10 mmoL/L, 1 to 5 mmoL/L or 1 to 3 mmoL/L, or 100 to 1000 mg/L or 200 to 500 mg/L.
(ix) antioxidant vitamins or their water-soluble analogs/derivatives, in the following.
   Each in the following take a form of salt. In some cases, ix-1 may be omitted.
   ix-1. Ascorbic acid 0.03 to 0.3 mmoL/L, 0.05 to 0.2 mmoL/L or 0.05 to 0.15 mmoL/L, or 5 to 20 mg/L or 10 to 15 mg/L.
   ix-2. Vitamin E, trolox or other water-soluble vitamin E analog/derivatives 0.5-10 mM, 1-8 mM or 2-7 mM.
(x) Adenine or its salt or derivative 0.1-6 mM, 0.1-5 mM, 0.1-4.5 mM, 0.1-4.2 mM or 2-5 mM, and in some cases, 0.1-0.3 mM or 0.05-0.2 mM.
(xi) Other vitamins not mentioned above, or water-soluble analog derivatives thereof
   Each in the following may take a form of salt. And, out of ten compounds in the following, one to five compounds, one to four compounds, or one to three compounds may be omitted.
   xi-1. Biotin 0.03 to 0.25 µmoL/L, 0.05 to 0.2 µmoL/L or 0.1 to 0.15 µmoL/L, or 0.01 to 0.1 mg/L or 0.02 to 0.05 mg/L.
   xi-2. Vitamin B12 0.03 to 3 µmoL/L, 0.05 to 2 µmoL/L or 0.1 to 0.5 µmoL/L, or 0.1 to 0.8 mg/L or 0.2 to 0.6 mg/L.
   xi-3. Folic acid 0.2-2 µmoL/L, 0.3-1 µmoL/L or 0.5-0.9 µmoL/L, or 0.1-1 mg/L or 0.2-0.5 mg/L.
   xi-4. Niacinamide 0.5-8 µmoL/L, 1-6 µmoL/L or 1-4 µmoL/L, or 0.03-0.2 mg/L or 0.05-0.1 mg/L.
   xi-5. Riboflavin 0.03 to 0.3 µmoL/L, 0.04 to 0.2 µmoL/L or 0.05 to 0.15 µmoL/L, or 0.03 to 0.2 mg/L or 0.05 to 0.1 mg/L.
   xi-6. Choline 0.05 to 1 µmoL/L, 0.1 to 0.5 µmoL/L or 0.1 to 0.4 µmoL/L, or 0.1 to 1 mg/L or 0.2 to 0.5 mg/L.
   xi-7. Inositol 1-10 µmoL/L, 1-8 µmoL/L or 2-6 µmoL/L, or 0.03-0.2 mg/L or 0.05-0.1 mg/L.
   xi-8. Pantothenic acid 0.02 to 0.2 µmoL/L, 0.03 to 0.1 µmoL/L or 0.04 to 0.08 µmoL/L, or 0.1 to 1 mg/L or 0.2 to 0.5 mg/L.
   xi-9. Pyridoxal 0.5 to 3 µmo L/L, 0.1 to 2 µmo L/L or 1 to 2 µmo L/L, or 0.1 to 1 mg/L or 0.2 to 0.5 mg/L.
   xi-10. Thiamine 0.3 to 3 µmoL/L, 0.4 to 2 µmoL/L or 0.5 to 1.5 µmoL/L, or 0.1 to 1 mg/L or 0.2 to 0.5 mg/L.
(xii) Essential amino acids 50-200 mg/L or 80-150 mg/L in total.
   Amount of each component may be in a range of A/3 to 3A or of A/2 to 2A, where A is the value obtained by dividing by 3, the content shown in Table 2 or the value in "HTM-alpha" in Examples mentioned later. That is, amount of each component may be set in a range of 1/3 to 3 times, or in a range of 1/2 to 2 times, of the value in "HTM-alpha". The same applies to the dictated non-essential amino acids and the vitamins mentioned above.
   xii-1. Isoleucine
   xii-2. Leucine
   xii-3. Ricin
   xii-4. Methionine
   xii-5. Phenylalanine
   xii-6. Threonine
   xii-7. Tryptophan
   xii-8. Valine
   xii-9. Histidine
(xiii) Non-essential amino acids in the following, 100-500 mg/L or 150-400 mg/L in toral.
   Each in the following may take a form of salt. And, out of ten compounds in the following, one to five compounds, one to four compounds, or one to three compounds may be omitted.
   xiii-1. Glycine
   xiii-2. Alanine
   xiii-3. Arginine
   xiii-4. Asparagine
   xiii-5. Aspartic acid
   xiii-6. Cysteine
   xiii-7. Cystine
   xiii-8. Glutamic acid
   xiii-9. Glutamine
   xiii-10. Proline
(xiv) Potassium ion species 20-90 mmoL/L, 30-80 mmoL/L, 40-80 mmoL/L or 50-70 mmoL/L. Namely, 20 mmoL/L or more, 30 mmoL/L or more, 40 mmoL/L or more, or 50 mmoL/L or more, and less than 90 mmoL/L, less than 80 mmoL/L, or less than 70 mmoL/L.
(xv) Sodium ion species 20-90 mmoL/L, 30-80 mmoL/L, 40-80 mmoL/L or 50-70 mmoL/L. Namely, 20 mmoL/L or more, 30 mmoL/L or more, 40 mmoL/L or more, or 50 mmoL/L or more, and less than 90 mmoL/L, less than 80 mmoL/L, or less than 70 mmoL/L.

This non-freezing refrigerated storage liquid preferably contains the following (xvi). (xvi) Hydroxyethyl starch 10-80 g/L, 20-50 g/L or 20-40 g/L.

This non-freezing refrigerated storage liquid preferably contains at least one of the following (xvii) and (xviii).
(xvii) Polyvinyl alcohol 3-100g/L, 5-80g/L, 5-50g/L, 5-30g/L, 5-25g/L, or 5-20g/L.
(xviii) Mannitol 20-100 mmoL/L, 30-90 mmoL/L or 20-90 mmoL/L.

This non-freezing refrigerated storage liquid preferably contains at least one of the following (xix) and (xx).
(xix) Ribonucleoside 2-4 types Total 4-40 mg/L, 5-30 mg/L or 10-15 mg/L.
(xx) Deoxyribonucleosides 2-4 types Total 4-40 mg/L, 5-30 mg/L or 10-15 mg/L.
(xxi) Potassium dihydrogen phosphate or sodium dihydrogen phosphate 10-30 mmoL/L, 10-25 mmoL/L, or 15-25 mmoL/L.

Further, according to a preferred embodiment of the present invention, in term of molar ratio (ratio of Na/K ions), amount of the sodium ion species is, for example, 0.7 to 1.3 times, 0.8 to 1.2 times, or 0.9 to 1.1 times that of the potassium ion species. According to another preferred embodiment, amount of the sodium ion species may be, for example, 0.5 to 1.5 times, 0.5 to 1.3 times, or 0.6 to 1.2 times of the potassium ion species, in the term of molar ratio.

As described above, the non-freezing refrigerated storage liquid according to the preferred embodiment of the present invention contains trolox or other water-soluble vitamin analogs in an amount of 0.1 mM or more, 0.3 mM or more, 0.5 mM or more, 1 mM or more, 2 mM or more, 3 mM, 4 mM or more, or 5 mM or more; and 10 mM or less, 8 mM or less, or 6 mM or less.

As described above, the non-freezing refrigerated storage liquid according to the preferred embodiment of the present invention contains, in particular, adenine or a salt thereof at 0.1 mM or more, 0.3 mM or more, 0.5 mM or more, 1 mM or more, 2 mM or more, 3 mM or more; and 8 mM or less, 6 mM or less, 5 mM or less or 4 mM or less; and, for example, 0.1 to 4.2 mM or 1 to 4.2 mM. In particular, it is considered that the addition of adenine has a remarkable effect in preserving embryos and organoids in which cells are adhered to each other to form a three-dimensional mass. In addition, in preserving embryos and organoids, effects were observed even at relatively low concentrations, for example, 0.01 to 0.2 mM (10 to 200 µM) or 0.05 to 0.3 mM (5 to 300 µM). On the other hand, when culturing stem cells such as iPS cells without forming embryos or organoids, it is considers as particularly effective to add adenine at a concentration of, for example, 0.5 to 6 mM, particularly 1 to 5 mM or 2 to 4 mM.

The non-freezing refrigerated storage liquid according to the preferred embodiment of the present invention further comprises L-alanyl-L-glutamine, or other dipeptide substitute, or a salt thereof, at a concentration of 1 to 6 mM, 2 to 5 mM, or 3 to 5 mM.

The refrigerated storage liquid according to a preferred embodiment of the present invention is obtainable by mixing a UW solution having a composition shown in Table 1 with the MEM alpha medium having a composition shown in Table 2, so that amount of the UW solution is 1 to 3 times, preferably 1.5 to 2.5 times, more preferably 1.8 to 2.2 times of amount of the MEM alpha medium, in a volume ratio. The compositions of Tables 1 and 2 and the content of each component obtained from the above mixing ratio may be increased or decreased within a range of ± 50%, ± 40%, ± 30%, or ± 20% if necessary or appropriate. In addition, out of the components listed in Table 2, some components are omittable. Particularly omittable are: some of the non-essential amino acids (for example, 1 to 5 amino acids); some of the nucleosides (for example, 1 to 5 nucleosides); some vitamins (for example, 1 to 5 vitamins); some inorganic salts (for example, 1 to 3 salts), and phenol red.

A non-freezing refrigerated storage method according to a preferred embodiment of the present invention comprises: dispersing pluripotent cells in any of the above refrigerated storage liquids so that the cells are dispersed in a single cell state in a range of 1×10³ cells/mL to 1×10 cells/mL, or 1×10⁴ cells/mL to 1×10⁸ cells/mL; and storing in non-freezing refrigerated state for 1 to 10 days or 1 to 7 days, for example.

A non-freezing refrigerated storage method according to a preferred embodiment of the present invention comprises: seeding pluripotent cells into a culture container containing any of the above refrigerated storage liquids in a range of 1×10³ cells/cm² to 1×10⁹ cells/cm² or 1×10⁴ cells/cm² to 1×10⁸ cells/cm²; culturing for 2 to 6 days in such state; and then storing in non-freezing refrigerated state for 1 to 10 days or 1 to 7 days, for example.

According to the non-freezing refrigerated storage method according to a preferred embodiment, the storage is able to be made in a state cells are adhered to a culture medium, that is, in a normal culture state; in particular, the cells are able to be stored in a state the cells are remained to be attached to the culture medium throughout whole of the non-freezing refrigerated storage period including the arousal periods.

According to a preferred embodiment of the present invention, the pluripotent cells or stem cells to be stored in a non-freezing refrigerated state are pluripotent cells such as ES cells and induced pluripotent stem cells such as iPS cells, or hematopoietic stem cells, nerve stem cells, and liver stem cells, skin stem cells, reproductive stem cells, etc. In addition, the pluripotent cells to be stored in a non-freezing refrigerated state may be of human origin or originated from mammals such as primates, mice and guinea pigs.

Further, according to another embodiment, target of non-freezing refrigerated storage may be human or animal embryos, particularly embryos of domestic animals such as cows, horses, pigs and goats.

### Examples

### 1. Preparation of cells for non-freezing refrigerated storage experiments

A healthy human-derived human iPS cell line (253G1) obtained from the Center for iPS Cell Research and Application, Kyoto University was maintained and cultured under feeder-free conditions. For this culture, StemFit (registered trademark) AK02N (Ajinomoto), which is a commercially available medium for clinical research for human ES/iPS cells was used. When the cells reached 70-80% confluence on the 7th day after the start of the culture, the cells were enzymatically stripped off from a container by using 0.5X TrypLE^{®} Select, and then dispersed and washed. Subsequently, the cells were suspended in a new StemFit^{®} AK02N medium containing 10 µM Y27362 (Fujifilm Wako Pure Chemical Industries, Ltd.) as a ROCK inhibitor, and added with iMatrix-511 (MATRIXOME, Inc.) as a further cell culture substrate at 0.1 µg/cm². Then, the cells were seeded at a concentration of 1.3E3/cm² (1.3×10³/cm²). The day after the seeding, the medium was replaced with StemFit^{®} AK02N medium containing no Y27362, then the medium was replaced every 3 days and subcultured or tested on day 7.

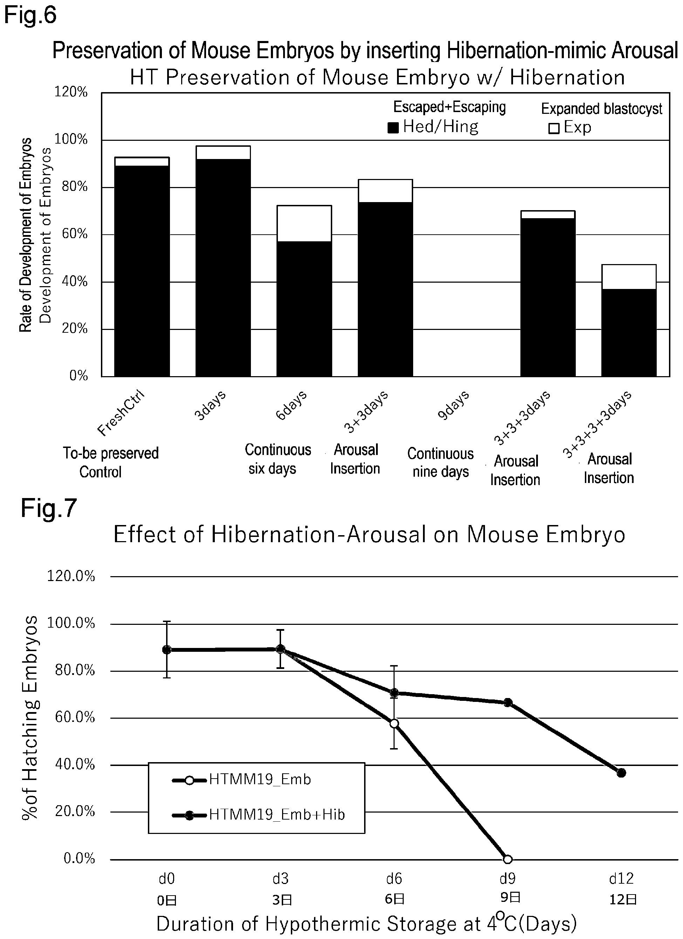

On the other hand, in order to try non-freezing refrigerated storage of mouse embryos (Fig. 6), in vitro fertilization was performed by ova and sperms from female and male mice (both ICR), and then embryos were cultured for 3.5 days (84 hours). Here, HTF (Fuji Film Wako Pure Chemical Industries, Ltd.) was used as the medium for insemination, and KSOM (Arc Resource) was used for culturing.

In addition, in present application, unless otherwise specified, culturing was carried out in a 37 °C incubator (5% CO₂).

### 2. Non-freezing refrigerated storage liquid

The following 3 types of refrigerated storage liquids were prepared. For convenience, they will be referred to as HTM-alpha, HTMX2C13, and HTMM19, respectively. Each of the refrigerated storage liquids was added on beforehand of being used, with trolox to becomes 5 mM, and was added with GlutaMAX (registered trademark, GIBCO) to become 5 times of standard concentration (x5; 10 mM).
- HTM-alpha: UW solution + MEM-alpha (12571--MEM alpha, nucleosides, Thermo Fisher Scientific), mixed volume ratio: 2/1.
- HTMx2c13: Modified from the HTM-alpha liquid. Mannitol 90mM and PVA 0.25% are added instead of hydroxyethyl starch. That is, from the composition of the UW solution in Table 1 below, a modified UW solution was prepared by omitting hydroxyethyl starch and by including 90mM mannitol and 0.25% polyvinyl alcohol; and thus modified UW solution and MEM-alpha (12571-MEM alpha) were mixed at a volume ratio of 2/1. The polyvinyl alcohol (PVA) used here was Merck #363146, which has a saponification degree of 99%, a weight average molecular weight (Mw) of 85,000 to 124,000, and a rotational viscosity of 28 to 32 mPa/sec for 4% aqueous solution at 20°C (28-32cP, 4% in H₂O(20°C)(lit.)).
- HTMM19: Modified from the HTM-alpha liquid. The MEM-alpha liquid was modified in respect of compositions of amino acids and vitamins to prepare modified MEM-alpha liquid; and the UW solution and thus modified MEM-alpha liquid were mixed at a volume ratio of 2:1 and used. The amino-acid composition and the vitamin composition, in the modified MEM-alpha solution used here, are shown respectively in Tables 4 and 5 shown later.

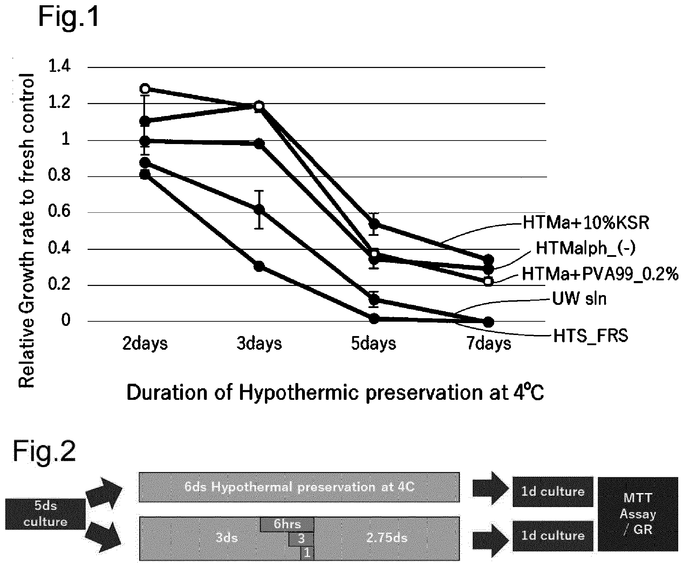

Further, in Reference Example shown in FIG. 1, the following two types of non-freezing storage liquids were also used. For convenience, they will be called HTMa+10%KSR and HTMa+PVA99_0.2%, respectively.
- HTMa+10%KSR: KnockOut ^{™} Serum Replacement (KSR) was added to the above HTM-alpha to a concentration of 10% by weight. KnockOut ^{™} Serum Replacement (KSR) used here is a serum-free supplement and serum replacement for culturing human and mouse pluripotent stem cells.
- HTMa+PVA99_0.2%: The above HTM-alpha was added with 0.2% by weight of polyvinyl alcohol (PVA). The polyvinyl alcohol used here was the same as that used in HTMx2c13 above. That is, the saponification degree is 99%, Mw is 85,000 to 124,000, and the rotational viscosity of a 4% aqueous solution at 20° C is 28 to 32 mPa/sec. Specifically, the polyvinyl alcohol used here was #363146 from Merck.

Any of the following two types of refrigerated storage liquids was used as the refrigerated storage liquid of the comparative example.
- UW solution (BELZER UW (registered trademark) COLD STORAGE LIQUID)
- HTS FRS (HypoThermosol^{®} FRS, BioLife Solutions)

The composition of the UW solution (BELZER UW (registered trademark) COLD STORAGE LIQUID) is as shown in the table below according to Non-Patent Document 1 above.

**[Table 1] Composition of UW solution**

| Ingredient | G/L | MMOL/L |
|---|---|---|
| Hydroxyethyl starch | 50.0 | NA |
| Lactobionic acid (lactone equivalent) | 35.83 | 105 |
| Potassium dihydrogen phosphate | 3.4 | 25 |
| Magnesium Sulfate Hydrate | 1.23 | 5 |
| Raffinose pentahydrate | 17.83 | 30 |
| Adenosine | 1.34 | 5 |
| Allopurinol | 0.136 | 1 |
| Total glutathione | 0.922 | 3 |
| Potassium hydroxide | 5.61 | 100 |
| Sodium hydroxide / hydrochloric acid | (for adjusting pH to 7.4) | |
| Water for injection | appropriate amount | |

Specifically, the MEM alpha medium (12571--MEM alpha, nucleosides, Thermo Fisher Scientific) used in "HTM-alpha" has the following composition (https://www.thermofisher.com/jp). /en/home/technical-resources/media-formulation.94.html).

**[Table 2] Composition of MEM alpha medium**

| **Components** | **Molecular Weight** | **Concentration (mg/L)** | **mM** |
|---|---|---|---|
| **Amino Acids** | | | |
| Glycine | 75.0 | 50.0 | 0.6666667 |
| L-Alanine | 89.0 | 25.0 | 0.28089887 |
| L-Arginine hydrochloride | 211.0 | 105.0 | 0.49763033 |
| L-Asparagine-H2O | 150.0 | 50.0 | 0.33333334 |
| L-Aspartic acid | 133.0 | 30.0 | 0.22556391 |
| L-Cysteine hydrochloride-H2O | 176.0 | 100.0 | 0.5681818 |
| L-Cystine 2HCl | 313.0 | 31.0 | 0.09904154 |
| L-Glutainic Acid | 147.0 | 75.0 | 0.5102041 |
| L-Glutamine | 146.0 | 292.0 | 2.0 |
| L-Histidine | 155.0 | 31.0 | 0.2 |
| L-Isoleucine | 131.0 | 52.4 | 0.4 |
| L-Leucine | 131.0 | 52.0 | 0.39694658 |
| L-Lysine | 183.0 | 73.0 | 0.3989071 |
| L-Methionine | 149.0 | 15.0 | 0. 10067114 |
| L-Phenylalanine | 165.0 | 32.0 | 0. 19393939 |
| L-Proline | 115.0 | 40.0 | 0.3478261 |
| L-Serine | 105.0 | 25.0 | 0. 23809524 |
| L-Threonine | 119.0 | 48.0 | 0.40336135 |
| L-Tryptophan | 204.0 | 10.0 | 0.04901961 |
| L-Tyrosine disodium salt | 225.0 | 52.0 | 0.23111111 |
| L-Valine | 117.0 | 46.0 | 0. 3931624 |

| **Vitamins** | | | |
|---|---|---|---|
| Ascorbic Acid | 176.0 | 50.0 | 0. 2840909 |
| Biotin | 244.0 | 0.1 | 4. 0983607E-4 |
| Choline chloride | 140.0 | 1.0 | 0. 007142857 |
| D-Calcium pantothenate | 477.0 | 1.0 | 0. 002096436 |
| Folic Acid | 441.0 | 1.0 | 0. 0022675737 |
| Niacinamide | 122.0 | 1.0 | 0.008196721 |
| Pyridoxal hydrochloride | 204.0 | 1.0 | 0.004901961 |
| Riboflavin | 376.0 | 0.1 | 2.6595744E-4 |
| Thiamine hydrochloride | 337.0 | 1.0 | 0.002967359 |
| Vitamin B12 | 1355.0 | 1.36 | 0.0010036901 |
| i-Inositol | 180.0 | 2.0 | 0.011111111 |

| **Inorganic Salts** | | | |
|---|---|---|---|
| Calcium Chloride (CaCl2) (anhyd.) | 111.0 | 200.0 | 1.8018018 |
| Magnesium Sulfate (MgSO4) (anhyd.) | 120.0 | 97.67 | 0.8139166 |
| Potassium Chloride (KCl) | 75.0 | 400.0 | 5. 3333335 |
| Sodium Bicarbonate (NaHCO3) | 84.0 | 2200.0 | 26.190475 |
| Sodium Chloride (NaCl) | 58.0 | 6800.0 | 117.24138 |
| Sodium Phosphate monobasic (NaH2PO4-H2O) | 138.0 | 140.0 | 1. 0144928 |

| **Ribonucleosides** | | | |
|---|---|---|---|
| Adenosine | 267.0 | 10.0 | 0.037453182 |
| Cytidine | 243.0 | 10.0 | 0.041152265 |
| Guanosine | 283.0 | 10.0 | 0.03533569 |
| Uridine | 244.0 | 10.0 | 0.040983606 |

| **Deoxyribonucleosides** | | | |
|---|---|---|---|
| 2' Deoxyadenosine | 251.0 | 10.0 | 0.03984064 |
| 2'Deoxycytidine HCl | 264.0 | 11.0 | 0.041666668 |
| 2'Deoxyguanosine | 267.0 | 10.0 | 0.037453182 |
| Thymidine | 242.0 | 10.0 | 0.041322313 |

| **Other Components** | | | |
|---|---|---|---|
| D-Glucose (Dextrose) | 180.0 | 1000.0 | 5.5555553 |
| Lipoic Acid | 206.0 | 0.2 | 9.708738E-4 |
| Phenol Red | 376.4 | 10.0 | 0.026567481 |
| Sodium Pyruvate | 110.0 | 110.0 | 1.0 |

GlutaMAX (registered trademark, GIBCO) added to "HTM-alpha" is a 200 mM solution ("GlutaMAX") in which L-alanyl-L-glutamine, as a dipeptide substitute for L-glutamine, is dissolved in physiological saline (0.85NNaCl). It is marketed as "GlutaMAX^{™} I (100 ×)". This was added into "HTM-alpha" so as to be diluted by 1/20 to become 10 mM (5 ×) when using.

With regard to the UW solution, HTM-alpha and HTMX2C13 in the above, and the HTS-FRS; sodium ion concentration, potassium ion concentration, and molar ratios between these two are listed in Table 3. It should be noted that StemFit (registered trademark) AK02N has the same ion concentration as MEM-alpha. In view of experimental results described later, it is considered that the molar ratio of Na⁺/K⁺ should be around 1 or a little smaller.

**[Table 3] Molar ratio of Na⁺/K⁺ in commercially available medium and media of Examples**

| | **UW solution** | **MEM-alpha** | **HTM-alpha, and HTMX2C13, HTMM19** | **HTS-FRS** |
|---|---|---|---|---|
| **Na⁺ (mM)** | 25.0 | 116.4 | 55.5 | 100.0 |
| **K⁺ (mM)** | 125.0 | 5.4 | 85.1 | 42.5 |
| **Na/K** | 0.2 : 1 | 21.7 : 1 | 0.7 : 1 | 2.3 : 1 |

As mentioned above, proteins, peptides, and growth factors are contained in StemFit^{®} AK02N, but not in MEM-alpha; and are therefore, not contained in HTM-alpha.

### 3. Refrigerated storage in adhesive state and subsequent MTT cell proliferation assay

Human iPS cells obtained in the same manner as in "1." above were used. However, at the final stage of culturing, the cells were seeded at a slightly lower concentration (1.0E3/cm²) than the maintenance culture of "1." above, and the day after this seeding, culture medium was replaced by StemFit^{®} AK02N medium containing no Y27362. Then, the medium was removed 4 days after the seeding.

Subsequently, the medium was replaced with each of the non-freezing refrigerated storage liquids that are described in "2." above; and the medium with the cells was left to stand for 3, 6 or 9 days under 4 °C. In other words, in a state the cells were aggregated and adhered to each other, the cells were stored in non-freezing refrigerated condition for prescribed periods.

The cells after the non-freezing refrigerated storage were lightly washed with culture medium, after the refrigerated storage medium was removed. Then, StemFit^{®} AK02N medium was added to the culture medium; and culturing was made for one day (24 hours).

At the end of this culture, cells were detached enzymatically (using 0.5X TrypLE^{®} Select), and dispersed, and cell proliferation was measured by MTT cell proliferation assay. Specifically, after adding Cosmo Bio Co., Ltd.'s "CytoSelect ^{™} MTT Cell Proliferation Assay" reagent, the cells were incubated at 37°C and 5% CO₂ for 3 hours, and solubilized in a surfactant solution for 3 hours. The absorbance at 540 nm was measured and evaluated by a colorimetric method.

Similarly, experiments were conducted using: the non-freezing refrigerated storage liquids HTMa+10%KSR and HTMa+PVA99_0.2% of the above-mentioned Modifications; and the non-freezing storage liquids of UW solution and HTS FRS of Comparative Examples.

### 4. Refrigerated storage using continuous refrigeration technique (conventional technique)

### (Reference Example)

Using the human IPS cells mentioned in "1." above and refrigeration storage liquids explained in "2." above, refrigeration storage at 4°C is made for 2 to 7 days as in "3." above. Results of the cell proliferation assay for such refrigerated storage are shown in FIG. 1.

According to the results shown in FIG. 1, it was known that: almost no damage was shown up to 3 days, but thereafter viability was rapidly decreased even with using any of: improved refrigerated preservation liquids of HTM-alpha and HTMX2C13, as well as their modifications (HTMa+10%KSR and HTMa+PVA99_0.2%) prepared by the present inventors. However, it was also confirmed that all of the improved refrigerated storage liquids had significantly greater preservation effects than the UW solution and HTS FRS.

Statistical processing in the Examples of the present application was performed using Graphpad Prism 5.04 using a 1-way ANOVA test.

### 5. Refrigerated storage of human stem cells adopting arousal-period insertion in a manner of hibernation (1)

FIG. 2 schematically shows the procedure for inserting the arousal and the subsequent cell proliferation assay according to the present invention. FIG. 1 shows the results when no arousal was inserted, as shown in the upper part of FIG. 2.

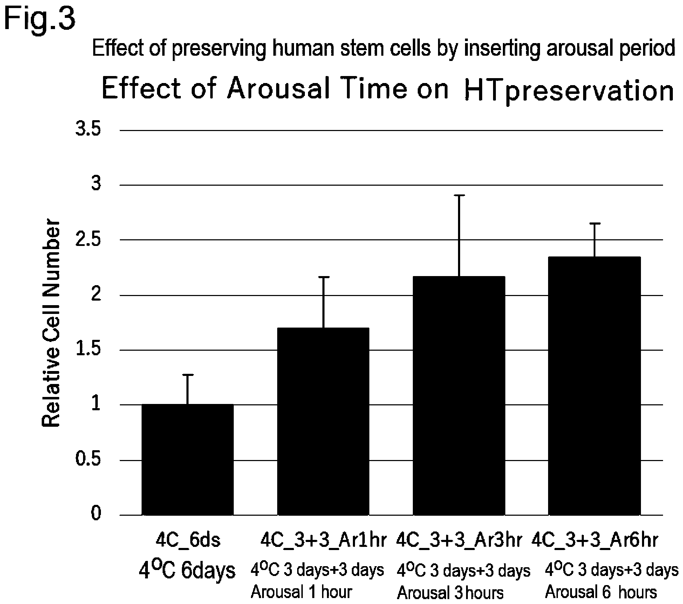

FIG. 3 shows effect of inserting an arousal period as shown in the lower part of FIG. 2. That is, 3 days (72 hours) after the start of refrigerated storage, the cells were transferred to a 37°C incubator and placed under culture conditions for 1 hour, 3 hours, or 6 hours. Then, the cells were returned to the refrigerator chamber at 4°C and kept for another 2.75 days (66 hours); then, after culturing for one day, a cell proliferation assay was performed.

As shown in FIG. 3, the cell proliferation rate was significantly increased by inserting the arousal period. Significant increase of the cell proliferation rate was shown when the arousal period was increased from one hour to three hours. Meanwhile, when the arousal period was increased from 3 to 6 hours, there seemed to be some further increase whereas it would be hard to say the increase was significant.

### 6. Refrigerated storage of human stem cells adopting arousal-period insertion in a manner of hibernation (2)

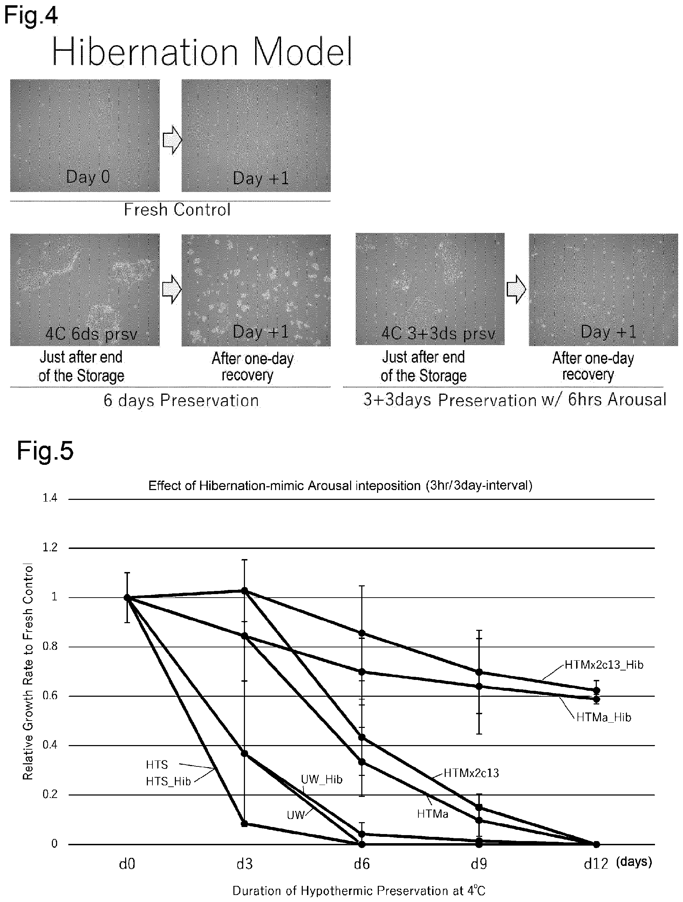

FIG. 4 shows, in gray scale, fluorescence micrographs showing dead cells immediately after the refrigerated storage mentioned in "5." above, and after the one-day recovery culture mentioned above, regarding the effect of arousal-period insertion in a manner of hibernation. Specifically, the cells were stained with Takara Bio Inc.'s Live or Dead ^{™} Dead Cell Staining Kit, and the stained areas are shown as white.

The upper left portion of FIG. 4 shows the state of the cells before being refrigerated when recovery culture was performed for one day in the same manner as above, but of course, no significant death of the cells was observed. The lower left portion of FIG. 4 shows the state of the cells after continuous refrigerated storage for 6 days without the arousal-period insertion. The area of dead cells was significantly larger after the 1 day of recovery culture than immediately after the refrigerated storage. On the other hand, when the 6-hour arousal period shown in the lower right of FIG. 4 was inserted, there was less cell death immediately after refrigerated storage than in the case of the lower-left portion of FIG. 4, after 1 day of recovery culture. The reduction in cell death was more pronounced.

### 7. Refrigerated storage of human stem cells adopting arousal-period insertion in a manner of hibernation (3)

The iPS cells in "1." above were incubated in each of the refrigerated storage liquids in "2." above, and arousal period of 3 hours was inserted every 3 days in exactly the same way as in FIGs. 2 to 3 above. Result of this is shown in FIG. 5, in comparison with the result when the insertion is not performed. Here, error bars in the line graph indicate the standard deviation based on 4 to 16 experiments.

What appears in upper-right end portion of FIG. 5 is the one in which improved refrigerated storage liquids (HTM-alpha and HTMx2c13) were used and an arousal period was inserted according to the present invention. These Examples of the present invention (HTM-a_Hib and HTMx2c13_Hib) showed a cell proliferation rate of about 60% or more as compared to that of to-be stored ones. On the other hand, even if the same refrigerated storage liquid is used, when the arousal period is not inserted, the cell proliferation rate after 6 and 9 days decreases significantly, as shown in bottom portion on right-hand half of FIG. 5. Thus, such way of storage was known to be not practical. Furthermore, after 12 days, almost no cell proliferation was observed. Furthermore, when using a conventional general refrigerated storage liquid (UW solution, HTS FRS), it was known to be difficult to preserve even for 3 days, and no effect of arousal insertion was observed.

### 8. Refrigerated storage of mouse embryos

As mentioned above, the blastocyst-stage embryos, which were 3.5 days after in vitro fertilization, were immediately used as they were, so as to be subjected to a refrigerated storage experiment. Specifically, these blastocyst-stage embryos were placed into plastic tubes, after replacing their culture medium with HTMM19, and then were left to stand at 4°C, for 3 days (72 hours), or for two times to four times of the three days; that is, for 6 days, 9 days and 12 days. The HTM-alphas used here was that supplemented with 5mM Trolox and with 5-times concentration of GlutaMAX (registered trademark) (x5; 10mM).

The embryos were further subj ected to culturing for two days. FIG. 6 shows rates of embryos that escaped the zona pellucida during the two-day culture period, and rates of embryo development. FIG. 6 also shows results for "Fresh_Control", or those not having been subjected to refrigeration storage, after being subjected to the culturing same with the above.

As shown in FIG. 6, relatively high rates of escaping from zona pellucida (solid-black part) and embryonic development (solid-black part + solid-white part) were observed after the refrigerated storage when arousal periods (3 hours each) were inserted at each three days, i.e., total of two times of the arousal periods into 9 days of refrigerated storage ("3+3+3days") and even into 12 days ("3+3+3+3days") of the refrigerated storage. In the column chart of FIG. 6, the solid-white part indicates an expanded blastocyst-stage embryos while "6 days" indicates a result after 6 days of refrigerated storage without inserting an arousal period. When refrigerated for 6 days with inserting an arousal period ("3+3days"), both of the rates of escaping from zona pellucida (Hed+Hing) and embryonic development ("Hed+Hing"+"Exp") were significantly higher than those when no insertion was performed ("6 days"). Furthermore, when the embryos were stored in a refrigerator for 9 days without an arousal period ("9 days"), no embryo development was observed.

In FIG. 6, "Hed+Hing" indicates total of: embryos having escaped from zona pellucida; and embryos escaping from the zona pellucida. Moreover, "Exp" indicates expanded blastocyst-stage embryos. Expanded blastocysts are meant to be in a state just before they become hatched blastocysts, and have a high probability of normally developing embryos, so we decided to include them in the embryonic development rate. Although data are not shown here, UW solution and HTS FRS showed significantly lower escape rates of 29.4% and 15.0%, respectively, in 3-day storage test plots.

FIG. 7 shows, in a form of a line chart, the rates of escaping from transparent body (solid-black part), which are shown in FIG. 6. Here, error bars in the line chart indicate standard deviations based on two to six repeats of the experiments.

Tables 4 and 5 show the amino acid composition and vitamin composition of the modified MEM-alpha liquid having been used to obtain the refrigerated storage liquid HTMM19 (2:1 mixture of UW solution and modified MEM-alpha solution). As seen by comparing these with the composition of the MEM alpha medium in Table 3, some amino acids and vitamins are omitted in the modified MEM-alpha liquid.

**[Table 4] Amino acid composition of modified MEM-alpha solution**

| Components | Molecular Weight | Concentration (mg/L) | mM |
|---|---|---|---|
| Glycine | 75 | 3.75 | 0.05 |
| L-Alanine | 89 | 4.45 | 0.05 |
| L-Arginine hydrochloride | 211 | 252.8 | 1.20 |
| L-Asparagine | 132 | 6.6 | 0.05 |
| L-Aspartic acid | 133 | 6.65 | 0.05 |
| L-Cystine | 240 | 48 | 0.20 |
| L-Glutamic Acid | 147 | 73.5 | 0.50 |
| L-Histidine hydrochloride-H₂O | 210 | 84 | 0.40 |
| L-Isoleucine | 131 | 104.8 | 0.80 |
| L-Leucine | 131 | 104.8 | 0.80 |
| L-Lysine hydrochloride | 183 | 145 | 0.79 |
| L-Methionine | 149 | 30.2 | 0.20 |
| L-Phenylalanine | 165 | 66 | 0.40 |
| L-Proline | 115 | 5.75 | 0.05 |
| L-Serine | 105 | 5.25 | 0.05 |
| L-Threonine | 119 | 95.2 | 0.80 |
| L-Tryptophan | 204 | 20.4 | 0.10 |
| L-Tyrosine | 181 | 72 | 0.40 |
| L-Valine | 117 | 93.6 | 0.80 |

**[Table 5] Vitamin composition of modified MEM-alpha liquid**

| Components | Molecular Weight | Concentration (mg/L) | mM |
|---|---|---|---|
| Choline chloride | 140 | 0.5 | 0.021 |
| D-Calcium pantothenate | 477 | 0.5 | 0.006 |
| Folic Acid | 441 | 0.5 | 0.007 |
| Nicotinamide | 122 | 0.5 | 0.025 |
| Pyridoxal hydrochloride | 204 | 0.5 | 0.015 |
| Riboflavin | 376 | 0.05 | 0.001 |
| Thiamine hydrochloride | 337 | 0.5 | 0.009 |
| i-Inositol | 180 | 1 | 0.033 |

While non-freezing refrigerated storage technology for iPS cells has a limited storage period as compared to freezing preservation, present technology has a very wide range of applications such as: storage in a cultured state (as adhered) as are; storage and transport of differentiation-induced organoids, which are not able to be frozen; or on-demand cell supply in drug discovery research. Therefore, present technology, which enables preserving human iPS cells, would be extremely important.

Effects have been shown in non-freezing refrigerated preservation of mouse embryos by using the cell cryopreservation medium "HTM-alpha" of the embodiment or the like; and thus presumably, effects will be shown in non-freezing refrigerated preservation of: embryotic or non-embryotic stem cells of human and various animals, or tissues comprising such stem cells. Moreover, effects have been shown in non-freezing refrigerated preservation of stem cells, which are generally difficult to be preserved; and thus presumably, effects will be shown in non-freezing refrigerated preservation of: other cells and tissues, as well as organs and parts thereof.

## Claims

1. A refrigerated storage method for storing human or animal stem cells or embryos in a refrigerated storage liquid, in a non-frozen state,
wherein arousal period of maintaining a temperature of from 20°C to 37°C for 0.5 to 8 hours, is inserted into each storage period of two days to eight days;
the refrigerated storage liquid comprising:
potassium ion species in a range of 30 to 80 mmoL/L;
sodium ion species in a range of 30 to 80 mmoL/L, wherein ion-based molar ratio of sodium ion species to potassium ion species (ratio of Na⁺ to K⁺) is in a range of 0.5 to 1.3;
Trolox or its analog at a concentration of 0.5 to 8 mM;
adenine or a salt or derivative thereof at a concentration of 0.1 to 4.2 mM;
all of or all but one, two or three kinds of essential amino acids, total content of which is 50 to 200 mg; and
non-essential amino acids, total content of which is 50 to 200 mg/L.

2. A refrigerated storage method for storing human or animal stem cells or embryos in a refrigerated storage liquid, in a non-frozen state,
wherein arousal period of maintaining a temperature of from 20°C to 37°C for 0.5 to 8 hours, is inserted into each storage period of two days to eight days;
the refrigerated storage liquid comprising:
(i) lactobionic acid or its salt in terms of lactone in a range of 30 to 100 mmoL/L,
(ii) raffinose hydrate in a range of 10 to 30 mmoL/L,
(iii) alloprinol in a range of 0.3 to 1 mmoL/L,
(iv) glutathione (total glutathione) in a range of 1 to 3mmoL/L,
(v) adenosine in a range of 2 to 10mmoL/L,
(vi) lipoic acid in a range of 0.1 to 0.5µmoL/L,
(vii) sodium pyruvate in a range of 0.1 to 0.7mmoL/L,
(viii) glucose in a range of 1 to 5mmoL/L,
(ix) ascorbic acid in a range of 0.03 to 0.3 mmoL/L and vitamin E or its water-soluble analog/derivative in a range of 0.5 to 8 mM,
(x) adenine or its salt or derivative in a range of 0.1 to 4.2 mM,
(xi) other vitamins or its water-soluble analog/derivatives, including folic acid, nicotine amide, riboflavin, B12, choline, inositol, pantothenic acid, pyridoxal phosphate and thiamine,
(xii) essential amino acids 50 to 200 mg/L in total,
(Xiii) non-essential amino acids, in a range of 100 to 500 mg/L in total,
(xiv) potassium ion species in a range of 30 to 80 mmoL/L, and
(xv) sodium ion species in a range of 20 to 90 mmoL/L.

3. The refrigerated storage method according to claim 1 or 2, wherein the refrigerated storage liquid further comprises:
20 to 50 g/L of hydroxyethyl starch,
5 to 50 g/L of polyvinyl alcohol, and/or
30 to 90 mmoL/L of mannitol.

4. The refrigerated storage liquid according to any one of claims 1 to 3, wherein the refrigerated storage liquid further comprise L-alanyl-L-glutamine or another dipeptide substitute, or a salt thereof, at a concentration of 2 to 5 mM.

5. A refrigerated storage method comprising: suspending or immersing human or animal iPS cells or other artificial stem cells, other stem cells, organoids obtained from them, or embryos in the refrigerated storage liquid according to any one of claims 1 to 4; and storing the cells in such suspended or immersed state, at 2 to 8 °C for 1 to 10 days.

6. A refrigerated storage liquid for storing human or animal stem cells or embryos in a non-frozen state,
wherein arousal period of maintaining a temperature of from 20°C to 37°C for 0.5 to 8 hours, is inserted into each storage period of two days to eight days;
the refrigerated storage liquid comprising:
potassium ion species in a range of 30 to 80 mmoL/L;
sodium ion species in a range of 30 to 80 mmoL/L, wherein ion-based molar ratio of sodium ion species to potassium ion species (ratio of Na⁺ to K⁺) is in a range of 0.5 to 1.3;
Trolox or its analog at a concentration of 0.5 to 8 mM;
adenine or a salt or derivative thereof at a concentration of 0.1 to 4.2 mM;
all of or all but one, two or three kinds of essential amino acids, total content of which is 50 to 200 mg; and
non-essential amino acids, total content of which is 50 to 200 mg/L.

7. A refrigerated storage liquid for storing human or animal stem cells or embryos in a non-frozen state,
wherein arousal period of maintaining a temperature of from 20°C to 37°C for 0.5 to 8 hours, is inserted into each storage period of two days to eight days;
the refrigerated storage liquid comprising:
(i) lactobionic acid or its salt in terms of lactone in a range of 30 to 100 mmoL/L,
(ii) raffinose hydrate in a range of 10 to 30 mmoL/L,
(iii) alloprinol in a range of 0.3 to 1 mmoL/L,
(iv) glutathione (total glutathione) in a range of 1 to 3mmoL/L,
(v) adenosine in a range of 2 to 10mmoL/L,
(vi) lipoic acid in a range of 0.1 to 0.5µmoL/L,
(vii) sodium pyruvate in a range of 0.1 to 0.7mmoL/L,
(viii) glucose in a range of 1 to 5mmoL/L,
(ix) ascorbic acid in a range of 0.03 to 0.3 mmoL/L and vitamin E or its water-soluble analog/derivative in a range of 0.5 to 8 mM,
(x) adenine or its salt or derivative in a range of 0.1 to 4.2 mM,
(xi) other vitamins or its water-soluble analog/derivatives, including folic acid, nicotine amide, riboflavin, B12, choline, inositol, pantothenic acid, pyridoxal phosphate and thiamine,
(xii) essential amino acids 50 to 200 mg/L in total,
(Xiii) non-essential amino acids, in a range of 100 to 500 mg/L in total,
(xiv) potassium ion species in a range of 30 to 80 mmoL/L, and
(xv) sodium ion species in a range of 20 to 90 mmoL/L.
